Europäisches Patentamt

European Patent Office

Office européen des brevets

(19)

(11) **EP 1 416 419 A2**

(12) **EUROPEAN PATENT APPLICATION**

(43) Date of publication:
**06.05.2004 Bulletin 2004/19**

(51) Int Cl.$^7$: **G06F 19/00**

(21) Application number: **03256568.1**

(22) Date of filing: **17.10.2003**

| | |
|---|---|
| (84) Designated Contracting States:<br>**AT BE BG CH CY CZ DE DK EE ES FI FR GB GR HU IE IT LI LU MC NL PT RO SE SI SK TR**<br>Designated Extension States:<br>**AL LT LV MK** | (72) Inventors:<br>• **Brackett, Cameron**<br>　**Naperville Illiniois 60564 (US)**<br>• **Anand, Virinchipuram Jagannathan**<br>　**Brookfield Wisconsin 53045 (US)** |
| (30) Priority: **22.10.2002 US 65466** | (74) Representative: **Goode, Ian Roy**<br>**London Patent Operation**<br>**General Electric International, Inc.**<br>**15 John Adam Street**<br>**London WC2N 6LU (GB)** |
| (71) Applicant: **GE Medical Systems Information Technologies, Inc.**<br>**Milwaukee, Wisconsin 53223 (US)** | |

(54) **Method, system, computer product and encoding format for creating anonymity in collecting patient data**

(57)　A method for creating anonymity in collecting patient data. The method comprises receiving a medical report for a patient including patient identification data. A patient record (224) is searched for an anonymous patient identifier (218) (APID) corresponding to the patient. The search returns the APID (218) in response to locating it and returns a null value in response to not locating an APID (218). If the search returns a null value, an APID (218) corresponding to the patient is created. The APID (218) is added to the medical report and the patient identification data is removed from the medical report. The medical report is transmitted to a data repository in response to removing the patient identification data.

## FIG. 2

## Description

The present disclosure relates generally to a method for creating anonymity in collecting patient data and in particular, to a method for creating anonymity in collecting patient medical data for use in public data mining.

Hospitals typically utilize computer systems to manage the various departments within a hospital and data about each patient is collected by a variety of computer systems. For example, a patient may be admitted to the hospital for a Transthoracic Echo (TTE). Information about the patient (e.g., demographics and insurance) could be obtained by the hospital information system (HIS) and stored on a patient record. This information could then be passed to the cardiology department system (commonly known as the cardio vascular information system, or CVIS). Typically the CVIS is a product of one company, while the HIS is the product of another company. As a result, the database between the two will be different. Further, they will capture/retain and send different levels of granularity in the data. Once the patient information has been received by the CVIS, the patient can be scheduled for a TTE in the echo lab. Next, the TTE is performed by the sonographer. Images and measurements are taken and sent to the CVIS server. The reading physician (e.g., an echocardiographer) sits down at a review station and pulls the patient's TTE study. The echocardiographer then begins to review the images and measurements and creates a complete medical report on the study. When the echocardiographer completes the medical report, the report is sent to the CVIS server where it is stored and associated with the patient through patient identification data. This completed medical report is an example of the kind of report that could be sent to a data repository for public data mining.

Today, medical device manufacturers and drug companies face an ever-growing challenge in collecting clinical data on the real-life utilization of their products. As patient medical reports are becoming computerized, the ability to obtain real-life utilization data becomes easier. Further, the data is easier to combine and analyze (e.g., mine) for greater amounts of useful information. In order to accurately assess the impact of a particular drug or treatment on a patient it would be helpful to be able to analyze all medical reports relating to the particular patient. However, access to patient medical data is protected by federal law whenever a patient name is associated with the medical record. Therefore, data that is contained in a public database must not reveal the identity of the individual patients whose medical information is contained in the database. Because of this requirement, any data contained on a medical report or record that could aid in tracing the report back to a particular individual must be removed from the report prior to adding the report to a data repository for public data mining. Removing data that can be used to trace back to an individual can make it impossible to group and analyze all medical reports relating to a particular patient.

One aspect of the invention is a method for creating anonymity in collecting patient data. The method comprises receiving a medical report for a patient including patient identification data. A patient record is searched for an anonymous patient identifier (APID) corresponding to the patient. The search returns the APID in response to locating it and returns a null value in response to not locating an APID. If the search returns a null value, an APID corresponding to the patient is created. The APID is added to the medical report and the patient identification data is removed from the medical report. The medical report is transmitted to a data repository in response to removing the patient identification data.

Another aspect of the invention is a method for creating anonymity in collecting patient data. The method comprises receiving a medical report for a patient including patient identification data. A patient record is searched for an APID corresponding to the patient. The search returns the APID in response to locating it and returns a null value in response to not locating an APID. If the search returns a null value, an APID corresponding to the patient is created. The creating includes receiving a media access control (MAC) address and applying a first linear transformation matrix to the MAC address, resulting in a transformed MAC address. The creating also includes receiving a date/time and applying a second linear transformation matrix to the date/time, resulting in a transformed date/time. Further, the creating includes receiving an anonymity supplement and applying a third linear transformation matrix to the anonymity supplement, resulting in a transformed anonymity supplement. The transformed MAC address, transformed date/time and transformed anonymity supplement are concatenated resulting in the APID. Finally, the creating includes encrypting the APID and storing the encrypted APID in the patient record. The method for creating anonymity in collecting patient data further comprises adding the APID to the medical report and the patient identification data is removed from the medical report. The medical report is transmitted to a data repository in response to removing the patient identification data.

Another aspect of the invention is a system for creating anonymity in collecting patient data. The system comprises a network and a host system in communication with the network. The host system includes software to implement a method comprising receiving a medical report for a patient including patient identification data. A patient record is searched for an APID corresponding to the patient. The search returns the APID in response to locating it and returns a null value in response to not locating an APID. If the search returns a null value, an APID corresponding to the patient is created. The APID is added to the medical report and the patient identification data is removed from the medical report. The medical report is transmitted to a data repository in response to removing the patient identification data.

A further aspect of the invention is a computer program product for creating anonymity in collecting patient data. The computer program product comprises a storage medium readable by a processing circuit and storing instructions for execution by the processing circuit for receiving a medical report for a patient including patient identification data. A patient record is searched for an APID corresponding to the patient. The search returns the APID in response to locating it and returns a null value in response to not locating an APID. If the search returns a null value, an APID corresponding to the patient is created. The APID is added to the medical report and the patient identification data is removed from the medical report. The medical report is transmitted to a data repository in response to removing the patient identification data.

A further aspect of the invention is an encoding format for creating anonymity in collecting patient data. The format comprises a unique system identifier and a patient identifier that includes a date/time component and an additional component to ensure uniqueness within the system. The APID is stored in an encrypted format on a patient record and the APID is stored in an unencrypted format on a medical report.

Embodiments of the invention will now be described, by way of example, with reference to the accompanying drawings, in which:

FIG. 1 is a flowchart of an exemplary process for creating anonymity in collecting patient data;

FIG. 2 is flowchart of an exemplary process for creating an anonymous patient identifier; and

FIG. 3 depicts the transformation of a data stream into an exemplary encoded anonymous patient identifier.

An embodiment of the present invention provides an anonymous method of collecting patient medical records generated by a medical site, such as a hospital, for use in public data mining. An embodiment of the present invention can facilitate the mining of medical outcomes and diagnosis and enhance patient care. The collection of this information maintains grouping of patient studies through the creation and use of an anonymous patient identifier (APID). For example, if John Doe had ten studies during one or more visits, then all of the studies for John Doe will be grouped under a single APID. The structure of the APID is designed to prevent duplication with other APIDs generated at the same or other sites using the same process. This is accomplished by creating a unique surrogate identifer, the APID, that is assigned to each patient. When a patient is created (manually or received by another system) in the departmental system, a unique APID is created for that patient. The APID is automatically encrypted and stored with the patient data in the patient record section of the database. The APID is encrypted by a separate application that has no access other than the APID encryption. Each time a patient's study report is copied for collection, the patient identification items (e.g., patient name and patient ID) are removed and replaced by the APID.

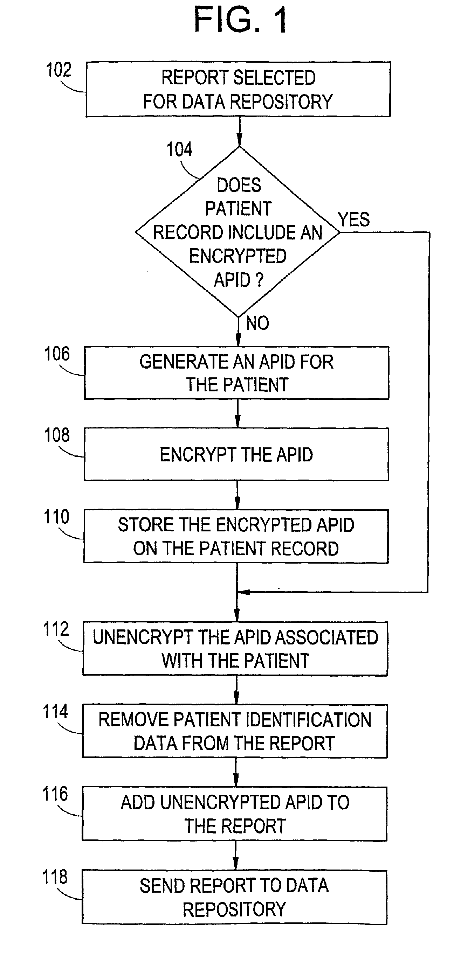

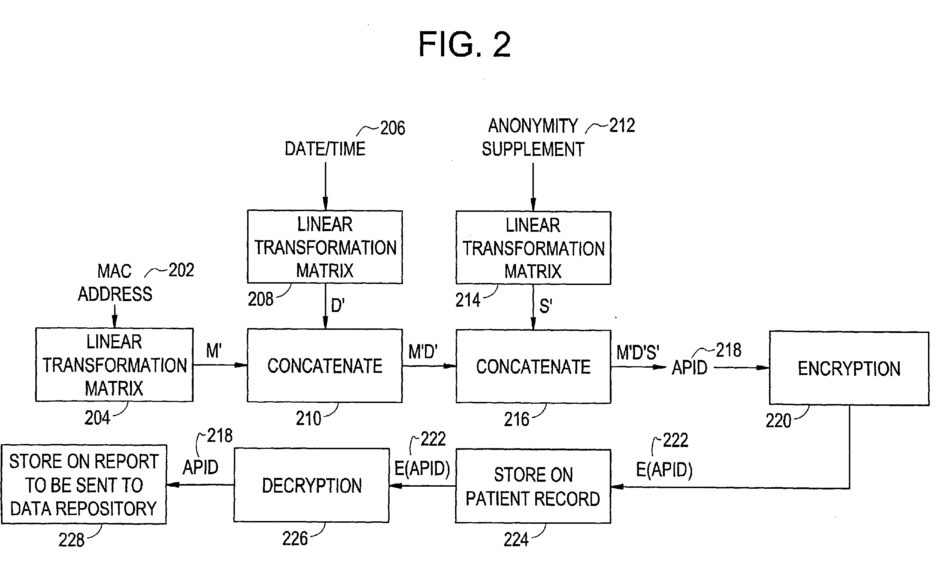

FIG. 1 is a flowchart of an exemplary process for creating anonymity in collecting patient data. At step 102, a report is selected to be sent to the data repository for use in public data mining. Report selection may be triggered when the report is completed, or alternatively, the database of reports may be periodically searched and reports in the database selected for the data repository based on pre-selected search criteria. Search criteria can include things like selecting all reports that have not been previously sent to the data repository or selecting only reports that relate to particular medical problems or treatments. At step 104, a check is made to determine if the patient record associated with the patient specified in the report already includes an encrypted APID that can be linked to the selected report. If the patient record includes an encrypted APID, processing continues at step 112. Otherwise, an encrypted APID must be created to correspond to the patient in the report. At step 106, an APID is generated for the patient. FIG. 2, discussed below, describes an exemplary embodiment of a process for creating an APID. At step 108, the APID is encrypted using any encryption software known in the art (e.g., PGP Corporation's PGP and RSA's BSAFE) At step 110, the encrypted APID is stored on the patient record.

Next, at step 112, the encrypted APID associated with the patient in the report selected for collection for the data repository is unencrypted using decryption software that corresponds to the encryption software utilized at step 108. In an exemplary embodiment, if the patient record did not include an encrypted APID, the unencrypted APID created in step 106 could be utilized and step 112 could be skipped. At step 114, patient identification data is removed from the report. Patient identification data includes any information or combination of information that could be used to identify a specific individual and can include name, social security number, insurance numbers and address. At step 116, the unencrypted APID is added to the report and at step 118, the report is sent to the data repository for use in public data mining.

FIG. 2 is a flowchart of an exemplary embodiment for creating an APID 218 for use in an embodiment of the present invention. It is important that a unique APID 218 is created for each patient across all hospitals and data sources for the data repository. In an exemplary embodiment of the present invention the APID 218 includes three components: a media access control (MAC) address; a date/time and an anonymity supplement. As depicted in FIG. 2, the MAC address 202 is input to a first linear transformation matrix 204. The MAC address 202 is a unique number that is burned into an Ethernet or token ring adapter that distinguishes it from all other network cards. The use of the MAC address 202 can ensure uniqueness among the mutually exclusive systems, or hospitals, that may be utilized to collect data for the data repository. The linear transformation matrix can be any non-singular linear matrix. In an exemplary embodiment the non-singular matrix is a three by three matrix, in another it is a one by three matrix. Using a three by three matrix as an example, the defined linear transformation is:

$$L:R^3 \rightarrow R^3 \text{ by } L(X) = AX$$

The output from the linear transformation matrix applied to the MAC address 202, the transformed MAC address, is denoted as M' in FIG. 2.

The second component to the APID 218 is the date/time 206 and it represents the date and time that the patient record was created in the system. In an exemplary embodiment, the date/time 206 component includes three subcomponents: date in "mmddyy" format, time in "hhmmss" format, and a constant digit. The date/time 206 component is utilized in order to provide uniqueness within the hospital where the patient was treated. The date/time 206 component also goes through a second linear transformation matrix 208 to prevent the ability to guess all of the patients for a given date and time. This second linear transformation matrix 208 may be the same or different than the first first linear transformation matrix 204 applied to the MAC address 202. In an exemplary embodiment, the second linear transformation matrix 208 can be any non-singular linear matrix such as a three by three matrix or a four by four matrix. Using a three by three matrix as an example, the defined linear transformation is:

$$L:R^3 \rightarrow R^3 \text{ by } L(X) = AX$$

The output from the linear transformation 208, the transformed date/time, is denoted as D' in FIG. 2. Both M' and D' are input to a concatenate function 210 to be concatenated together and the resulting output is denoted as M'D'.

The third component to the APID 218 is the anonymity supplement 212 component. The anonymity supplement 212 component includes three subcomponents: a random number between 1 and 1000, a rotating number between 20 and 40, and a constant. The rotating number subcomponent increments by 1 each time an anonymity supplement is created and once it gets to 40 it returns back to 20. The anonymity supplement 212 component is utilized in order to prevent the APID from being predicted by individuals trying to identify the individual patient being discussed in the report. It also is utilized to get around the case where two patients are admitted to the same hospital at exactly the same time. The anonymity supplement 212 component also goes through a third linear transformation matrix 214. This third linear transformation matrix 214 may be the same or different than the linear transformation matrices 204 208 applied to the MAC address 202 and date/time 206 components of the APID 218. In an exemplary embodiment, the third linear transformation matrix 214 can be any non-singular linear matrix such as a three by three matrix or a four by four matrix. Using a three by three matrix as an example, the defined linear transformation is:

$$L:R^3 \rightarrow R^3 \text{ by } L(X) = AX$$

The output from the third linear transformation matrix 214, the transformed anonymity supplement, is denoted as S' in FIG. 2. Both M'D' and S' are input to a concatenate function 216 to be concatenated together and the resulting output is denoted M'D'S' which is the APID 218.

The APID 218 is then sent through an encryption program 220 to create an encrypted APID 222 that is then stored on the patient record 224. When a request is made to attach an APID 218 to a report, a decryption program 226 that corresponds to the encryption program 220 is utilized to transform the encrypted APID 222 into an unencrypted APID 218. The unencrypted APID 218 is then stored on the report or linked to the report that is sent to the data repository for public data mining. The linear transformation matrices described above can differ between hospitals and are typically stable for ease in transformation. In an exemplary embodiment, the first first linear transformation matrix 204 utilized on the MAC address 202 is the same for all implementations and the other linear transformation matrices 208 214 differ between hospitals and are modified by product upgrades.

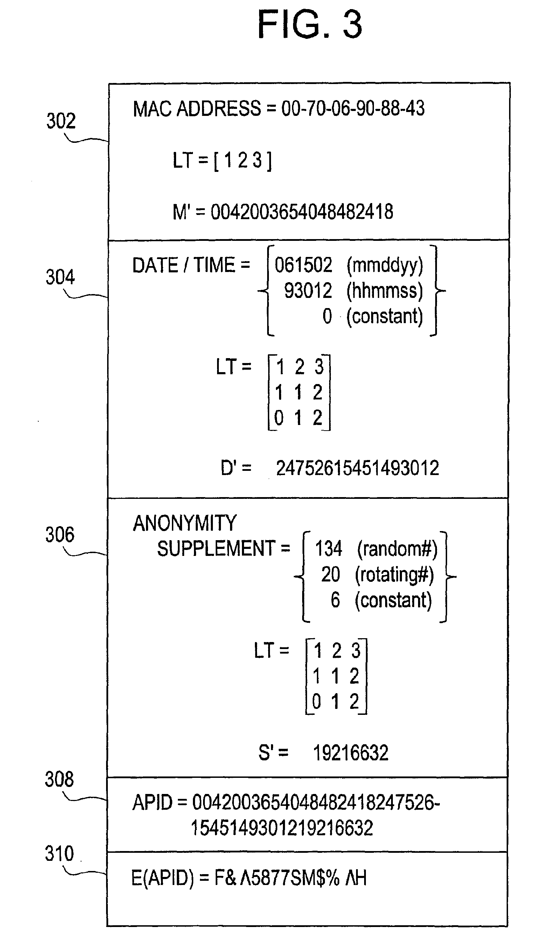

FIG. 3 depicts the transformation of a data stream into an exemplary encoded APID 218. The data contained in box 302 is a sample MAC address 202, a sample first first linear transformation matrix 204, "LT", and the transformed MAC address, M'. Box 304 includes a sample date/time 206, a sample second linear transformation matrix 208, "LT", and the transformed date/time, D'. Box 306 includes a sample anonymity supplement 212, a sample third linear transformation matrix 214, "LT", and the transformed anonymity supplement, S'. Box 308 contains the resulting APID 218 which is created by concatenating the three values: M', D' and S' together. Box 310 contains an example of an encrypted APID 222. The data values and formats depicted in FIG. 3 are meant to be examples of one way to implement the creation of an APID 218 utilizing the present invention, each implementation will include different linear transformation matrices and may include different data formats for the date/time 206 and anonymity supplement 212 components. Further, the MAC address is utilized to uniquely identify a computer system and any identifier that also uniquely identifies a computer system may be used in place of the MAC address. In addition, any encryption software known in the art can be utilized with an embodiment of the present invention.

An embodiment of the present invention allows for the creation of a unique and anonymous patient identifier that is attached to a medical report for use in grouping reports relating to a single patient without revealing the identity of the patient. This can allow for a secure mechanism for gathering anonymous patient medical data for use in public data mining. By including the MAC address, an existing and unique alphanumeric value assigned to each system, in the APID 218, different mutually exclusive systems can be utilized to originate anonymous patient medical data without resulting in duplicate APIDs 218 between systems. The use of the date/time 206 and the anonymity supplement 212 helps to ensure that the APIDs 218 are not duplicated within a single system. The ability to create a unique APID 218 can allow for more meaningful data mining because reports relating to a single patient can be reviewed and analyzed as a group. The ability to prevent a public data mine user from tracing a report back to an individual is important to protecting patient privacy. This is accomplished by sanitizing the medical reports by replacing the patient data with an APID 218 before transmission to a data repository and by creating an APID 218 that can not be traced back to an individual patient. Providing public data mining access to data reports, grouped by individual patient, can lead to better information for use by pharmaceutical companies and hospitals in improving medical procedures and products.

As described above, the embodiments of the invention may be embodied in the form of computer-implemented processes and apparatuses for practicing those processes. Embodiments of the invention may also be embodied in the form of computer program code containing instructions embodied in tangible media, such as floppy diskettes, CD-ROMs, hard drives, or any other computer-readable storage medium, wherein, when the computer program code is loaded into and executed by a computer, the computer becomes an apparatus for practicing the invention. An embodiment of the present invention can also be embodied in the form of computer program code, for example, whether stored in a storage medium, loaded into and/or executed by a computer, or transmitted over some transmission medium, such as over electrical wiring or cabling, through fiber optics, or via electromagnetic radiation, wherein, when the computer program code is loaded into and executed by a computer, the computer becomes an apparatus for practicing the invention. When implemented on a general-purpose microprocessor, the computer program code segments configure the microprocessor to create specific logic circuits.

For completeness, various aspects of the invention are set out in the following numbered clauses:

1. A method for creating anonymity in collecting patient data, the method comprising: receiving a medical report for a patient including patient identification data; searching a patient record (224) for an anonymous patient identifier (218) corresponding to said patient wherein said searching returns said anonymous patient identifier (218) in response to locating said anonymous patient identifier (218) and said searching returns a null value in response to not locating said anonymous patient identifier (218);
creating said anonymous patient identifier (218) corresponding to said patient if said searching returns said null value;
adding said anonymous patient identifier (218) to said medical report; removing said patient identification data from said medical report; and
transmitting said medical report to a data repository in response to said removing.

2. The method of clause 1 wherein said anonymous patient identifier (218) includes a linear transformation of a media access control address (202) component, a date/time (206) component and an anonymity supplement (212) component.

3. The method of clause 2 wherein said date/time (206) component includes a month, a day, a year, an hour, a minute, and a second corresponding to when said patient was first admitted.

4. The method of clause 2 wherein said anonymity supplement (212) component includes a rotating number that is incremented each time a new said anonymity supplement (212) is created.

5. The method of clause 2 wherein said linear transformation includes a non-singular matrix.

6. The method of clause 5 wherein said matrix is a three by three matrix.

7. The method of clause 5 wherein said matrix is a four by four matrix.

8. The method of clause 5 wherein said matrix is an n by n matrix.

9. The method of clause 1 wherein said patient identification data includes one of name, medical record number and social security number.

10. The method of clause 1 wherein said anonymous patient identifier (218) is in an encrypted format on said patient record (224).

11. The method of clause 1 wherein said anonymous patient identifier (218) is in an unencrypted format on said medical report.

12. The method of clause 1 wherein said creating said anonymous patient identifier (218) includes:

receiving a media access control address (202);
applying a first linear transformation matrix (204) to said media access control address (202) resulting in a transformed media access control address;
receiving a date/time;
applying a second linear transformation matrix (208) to said date/time (206) resulting in a transformed date/time;
receiving an anonymity supplement (212); applying a third linear transformation matrix (214) to said anonymity supplement (212) resulting in a transformed anonymity supplement;
concatenating said transformed media access control address, said transformed date/time and said transformed anonymity supplement (212) resulting in said anonymous patient identifier (222);
encrypting said anonymous patient identifier (218) resulting in an encrypted anonymous patient identifier (222); and
storing said encrypted anonymous patient identifier (218) in said patient record (224).

13. The method of clause 12 wherein said first linear transformation matrix (204), said second linear transformation matrix (208) and said third linear transformation matrix (214) are the same matrices.

14. The method of clause 12 wherein two of said first linear transformation matrix (204), said second linear transformation matrix (208) and said third linear transformation matrix (214) are different matrices.

15. A method for creating anonymity in collecting patient data, the method comprising:

receiving a medical report for a patient including patient identification data;
searching a patient record (224) for an anonymous patient identifier (218) corresponding to said patient wherein said searching returns said anonymous patient identifier (218) in response to locating said anonymous patient identifier (218) and said searching returns a null value in response to not locating said anonymous patient identifier (218);
creating said anonymous patient identifier (218) corresponding to said patient if said searching returns said null value, wherein said creating includes:

receiving a media access control address (202);
applying a first linear transformation matrix (204) to said media access control address (202) resulting in a transformed media access control address;
receiving a date/time (206);
applying a second linear transformation matrix (208) to said date/time (206) resulting in a transformed date/time;
receiving an anonymity supplement (212);
applying a third linear transformation matrix (214) to said anonymity supplement (212) resulting in a transformed anonymity supplement;
concatenating said transformed media access control address, said transformed date/time and said transformed anonymity supplement resulting in said anonymous patient identifier (218);
encrypting said anonymous patient identifier (218) resulting in an encrypted anonymous patient identifier (222); and
storing said encrypted anonymous patient identifier (222) in said patient record (224).;
adding said anonymous patient identifier (218) to said medical report; removing said patient identification data from said medical report; and
transmitting said medical report to a data repository in response to said removing.

16. A system for creating anonymity in collecting patient data, the system comprising:

a network; and
a host system in communication with said network, said host system including software to implement the method comprising:

receiving over said network a medical report for a patient including patient identification data;
searching a patient record (224) for an anonymous patient identifier (218) corresponding to said patient wherein said searching returns said anonymous patient identifier (218) in response to locating said anonymous patient identifier (218) and said searching returns a null value in response to not locating said anonymous patient identifier (218);
creating said anonymous patient identifier (218) corresponding to said patient if said searching returns said null value;
adding said anonymous patient identifier (218) to said medical report;

removing said patient identification data from said medical report; and

transmitting said medical report to a data repository over said network in response to said removing.

17. The system of clause 16 wherein said network is an Internet.

18. The system of clause 16 wherein said network is an intranet.

19. The system of clause 16 wherein said creating said anonymous patient identifier (218) includes:

receiving a media access control address;

applying a first linear transformation matrix (204) to said media access control address (202) resulting in a transformed media access control address;

receiving a date/time (206);

applying a second linear transformation matrix (208) to said date/time (206) resulting in a transformed date/time;

receiving an anonymity supplement (212);

applying a third linear transformation matrix (214) to said anonymity supplement (212) resulting in a transformed anonymity supplement;

concatenating said transformed media access control address, said transformed date/time and said transformed anonymity supplement resulting in said anonymous patient identifier (218);

encrypting said anonymous. patient identifier (218) resulting in an encrypted anonymous patient identifier (218); and

storing said encrypted anonymous patient identifier (222) in said patient record (224).

20. A computer program product for creating anonymity in collecting patient data, the product comprising:

a storage medium readable by a processing circuit and storing instructions for execution by the processing circuit for:

receiving a medical report for a patient including patient identification data;

searching a patient record (224) for an anonymous patient identifier (218) corresponding to said patient wherein said searching returns said anonymous patient identifier (218) in response to locating said anonymous patient identifier (218) and said searching returns a null value in response to not locating said anonymous patient identifier (218);

creating said anonymous patient identifier (218) corresponding to said patient if said searching returns said null value;

adding said anonymous patient identifier (218) to said medical report;

removing said patient identification data from said medical report; and

transmitting said medical report to a data repository in response to said removing.

21. The computer program product of clause 20 wherein said creating said anonymous patient identifier (218) includes:

receiving a media access control address (202);

applying a first linear transformation matrix (204) to said media access control address (202) resulting in a transformed media access control address;

receiving a date/time (206);

applying a second linear transformation matrix (208) to said date/time (206) resulting in a transformed date/time;

receiving an anonymity supplement (212);

applying a third linear transformation matrix (214) to said anonymity supplement (212) resulting in a transformed anonymity supplement;

concatenating said transformed media access control address, said transformed date/time and said transformed anonymity supplement resulting in said anonymous patient identifier (218);

encrypting said anonymous patient identifier (218) resulting in an encrypted anonymous patient identifier (222); and

storing said encrypted anonymous patient identifier (218) in said patient record (224).

22. An anonymous patient identifier (218) encoding format for creating anonymity in collecting patient data, the format comprising:

a unique system identifier;

a patient identifier including a date/time (206) component and an additional component to ensure uniqueness within said system; and wherein:

said anonymous patient identifier (218) is stored in an encrypted format on a patient record (224); and

said anonymous patient identifier (218) is stored in an unencrypted format on a medical report.

23. The encoding format of clause 22 wherein said unique system identifier is a linear transformation applied to a media access control address (202).

24. The encoding format of clause 22 wherein said date/time (206) component is a linear transformation applied to the concatenation of a month, day, year, hour, minute and second corresponding to when said patient was first admitted.

25. The encoding format of clause 22 wherein said additional component includes a linear transformation applied to the concatenation of a rotating number that is incremented each time a new said anonymity supplement (212) is created.

## Claims

1. A method for creating anonymity in collecting patient data, the method comprising: receiving a medical report for a patient including patient identification data; searching a patient record (224) for an anonymous patient identifier (218) corresponding to said patient wherein said searching returns said anonymous patient identifier (218) in response to locating said anonymous patient identifier (218) and said searching returns a null value in response to not locating said anonymous patient identifier (218);

creating said anonymous patient identifier (218) corresponding to said patient if said searching returns said null value;

adding said anonymous patient identifier (218) to said medical report; removing said patient identification data from said medical report; and

transmitting said medical report to a data repository in response to said removing.

2. The method of claim 1 wherein said anonymous patient identifier (218) includes a linear transformation of a media access control address (202) component, a date/time (206) component and an anonymity supplement (212) component.

3. The method of claim 2 wherein said date/time (206) component includes a month, a day, a year, an hour, a minute, and a second corresponding to when said patient was first admitted.

4. The method of claim 2 wherein said anonymity supplement (212) component includes a rotating number that is incremented each time a new said anonymity supplement (212) is created.

5. The method of claim 2 wherein said linear transformation includes a non-singular matrix.

6. The method of claim 1 wherein said creating said anonymous patient identifier (218) includes:

receiving a media access control address (202);
applying a first linear transformation matrix (204) to said media access control address (202) resulting in a transformed media access control address;
receiving a date/time;
applying a second linear transformation matrix (208) to said date/time (206) resulting in a transformed date/time;
receiving an anonymity supplement (212); applying a third linear transformation matrix (214) to said anonymity supplement (212) resulting in a transformed anonymity supplement;
concatenating said transformed media access control address, said transformed date/time and said transformed anonymity supplement (212) resulting in said anonymous patient identifier (222);
encrypting said anonymous patient identifier (218) resulting in an encrypted anonymous patient identifier (222); and
storing said encrypted anonymous patient identifier (218) in said patient record (224).

7. A method for creating anonymity in collecting patient data, the method comprising:

receiving a medical report for a patient including patient identification data;
searching a patient record (224) for an anonymous patient identifier (218) corresponding to said patient wherein said searching returns said anonymous patient identifier (218) in response to locating said anonymous patient identifier (218) and said searching returns a null value in response to not locating said anonymous patient identifier (218);
creating said anonymous patient identifier (218) corresponding to said patient if said searching returns said null value, wherein said creating includes:

receiving a media access control address (202);
applying a first linear transformation matrix (204) to said media access control address (202) resulting in a transformed media access control address;
receiving a date/time (206);
applying a second linear transformation matrix (208) to said date/time (206) resulting in a transformed date/time;
receiving an anonymity supplement (212);
applying a third linear transformation matrix (214) to said anonymity supplement (212) resulting in a transformed anonymity supplement;

concatenating said transformed media access control address, said transformed date/time and said transformed anonymity supplement resulting in said anonymous patient identifier (218);

encrypting said anonymous patient identifier (218) resulting in an encrypted anonymous patient identifier (222); and

storing said encrypted anonymous patient identifier (222) in said patient record (224).; adding said anonymous patient identifier (218) to said medical report; removing said patient identification data from said medical report; and

transmitting said medical report to a data repository in response to said removing.

8. A system for creating anonymity in collecting patient data, the system comprising:

a network; and

a host system in communication with said network, said host system including software to implement the method comprising:

receiving over said network a medical report for a patient including patient identification data;

searching a patient record (224) for an anonymous patient identifier (218) corresponding to said patient wherein said searching returns said anonymous patient identifier (218) in response to locating said anonymous patient identifier (218) and said searching returns a null value in response to not locating said anonymous patient identifier (218);

creating said anonymous patient identifier (218) corresponding to said patient if said searching returns said null value;

adding said anonymous patient identifier (218) to said medical report;

removing said patient identification data from said medical report; and

transmitting said medical report to a data repository over said network in response to said removing.

9. A computer program product for creating anonymity in collecting patient data, the product comprising:

a storage medium readable by a processing circuit and storing instructions for execution by the processing circuit for:

receiving a medical report for a patient including patient identification data;

searching a patient record (224) for an anonymous patient identifier (218) corresponding to said patient wherein said searching returns said anonymous patient identifier (218) in response to locating said anonymous patient identifier (218) and said searching returns a null value in response to not locating said anonymous patient identifier (218);

creating said anonymous patient identifier (218) corresponding to said patient if said searching returns said null value;

adding said anonymous patient identifier (218) to said medical report;

removing said patient identification data from said medical report; and

transmitting said medical report to a data repository in response to said removing.

10. An anonymous patient identifier (218) encoding format for creating anonymity in collecting patient data, the format comprising:

a unique system identifier;

a patient identifier including a date/time (206) component and an additional component to ensure uniqueness within said system; and wherein:

said anonymous patient identifier (218) is stored in an encrypted format on a patient record (224); and

said anonymous patient identifier (218) is stored in an unencrypted format on a medical report.

# FIG. 1

102 — REPORT SELECTED
FOR DATA REPOSITORY

104 — DOES PATIENT RECORD INCLUDE AN ENCRYPTED APID ? — YES

NO

106 — GENERATE AN APID FOR THE PATIENT

108 — ENCRYPT THE APID

110 — STORE THE ENCRYPTED APID ON THE PATIENT RECORD

112 — UNENCRYPT THE APID ASSOCIATED WITH THE PATIENT

114 — REMOVE PATIENT IDENTIFICATION DATA FROM THE REPORT

116 — ADD UNENCRYPTED APID TO THE REPORT

118 — SEND REPORT TO DATA REPOSITORY

# FIG. 2

EP 1 416 419 A2

# FIG. 3

**302** — MAC ADDRESS = 00-70-06-90-88-43

LT = [ 1 2 3 ]

M' = 0042003654048482418

**304** — DATE / TIME = $\left\{\begin{array}{ll} 061502 & \text{(mmddyy)} \\ 93012 & \text{(hhmmss)} \\ 0 & \text{(constant)} \end{array}\right\}$

$$LT = \begin{bmatrix} 1 & 2 & 3 \\ 1 & 1 & 2 \\ 0 & 1 & 2 \end{bmatrix}$$

D' = 24752615451493012

**306** — ANONYMITY
SUPPLEMENT = $\left\{\begin{array}{ll} 134 & \text{(random\#)} \\ 20 & \text{(rotating\#)} \\ 6 & \text{(constant)} \end{array}\right\}$

$$LT = \begin{bmatrix} 1 & 2 & 3 \\ 1 & 1 & 2 \\ 0 & 1 & 2 \end{bmatrix}$$

S' = 19216632

**308** — APID = 0042003654048482418247526-
1545149301219216632

**310** — E(APID) = F& Λ5877SM$% ΛH